# EUROPEAN PATENT APPLICATION

(11) **EP 4 235 686 A1**
(43) Date of publication of application: **30.08.2023**
(21) Application number: 22158151.5
(22) Date of filing: 23.02.2022
(51) Int. Cl.: G16H 30/40, G06T 7/00, A61B 6/00, A61B 34/00, A61N 5/00, G16H 20/40, G16H 30/20, G16H 40/63, A61B 8/00

(54) **REGION OF INTEREST INDICATION FOR LIVE MEDICAL IMAGES**

(71) Applicant: Koninklijke Philips N.V., 5656 AG Eindhoven (NL)
(72) Inventor: VYAPARLA, Sreedhar Reddy, Eindhoven (NL); CHAO, Pei Yin, Eindhoven (NL)
(74) Representative: Philips Intellectual Property & Standards

(57) **Abstract**

A system and a computer-implemented method is provided for displaying live medical images of a subject. It comprises receiving a reference medical image and a live medical image of the subject. The reference image contains at least one marked position. Angle and zoom characteristics of the reference image and the live image are obtained. The method further comprises checking whether the reference image and the live image align based on their angle and zoom characteristics. If the reference image and the live image align, the reference image and live image are displayed to the user and at least one guideline is overlaid on top of the reference image and the live image. The guideline crosses the at least one marked position on the reference image.

## Description

### FIELD OF THE INVENTION

The invention relates to live medical images, and to a method and system for assisting the interpretation of such medical images. In particular, the invention relates to live medical images supported by reference medical images.

### BACKGROUND OF THE INVENTION

Intravascular cardiologists and vascular surgeons use live X-ray images, i.e. fluoroscopy, supported by treatment planning images, to decide, guide and confirm treatment. In some systems, a co-registration feature displays different types of medical images. This, for example, enables them to map physiology or intravascular imaging data on an angiogram. Physiology and imaging-based treatment planning has proven to lead to better patient outcomes.

Despite the procedure time and additional X-ray radiation needed for defining an effective and accurate treatment strategy using the co-registration feature, the actual treatment itself can still be inaccurate because physicians need to translate positions on a static reference image to a moving fluoroscopy image using the bare eye. This can be especially challenging when the reference image and fluoroscopy image aren't positioned side by side, don't match in size, don't match in angulation and or don't match in magnification.

### SUMMARY OF THE INVENTION

The invention is defined by the claims.

According to examples in accordance with an aspect of the invention, there is provided a computer-implemented method for displaying live medical images of a subject, the method comprising:
receiving a reference medical image and a live medical image of the subject, wherein the reference image comprises at least one marked position;
obtaining angle and magnification characteristics of the reference image and the live image;
verifying whether the reference image and the live image are aligned based on their angle and magnification characteristics; and
if the reference image and the live image are aligned:
   displaying, to a user, the reference image and the live image; and
   overlaying at least one guideline on top of the reference image and the live image, wherein the guideline crosses the at least one marked position on the reference image.

This enables the user to determine the corresponding points between a reference image and the live image. The analysis of the image angle and the magnification, i.e. zoom, enables the method to check whether the reference image and the live image align and, if they do align, a guideline or reference-line can be overlaid on both such that any point (e.g. at a particular y axis position) of the reference image can be easily identified on the live image.

The invention thereby seeks to help interventional cardiologists and vascular surgeons to make more effective use of their treatment plans by aligning and positioning the reference image and live medical image side by side during treatment and adding visual guides on top of the two images that make it easier to correlate positions between the two images.

Treatment success will rely less on the ability of physicians to match positions between a static and moving image using the bare eye, thereby the region of interest identification and indication for live medical images is improved.

If the reference image and the live image do not align, the method may further comprise notifying the user that the images do not align. The method may comprise providing the user with an option to perform realignment of the live image with the reference image.

Notifying the user that they do not align allows the user to adapt the live image such that it does align with the reference image. This is particularly important when the reference image and the live image slightly misalign but may look like they align.

The method may further comprise displaying a plurality of co-registration images to the user and obtaining a user selected reference image from the plurality of co-registration images.

This enables the user to choose which reference image they would like to use. This allows reference images to be chosen which may be clearer or contain better distinction of points of interest (e.g. where the user may want to place the marked positions).

The method may further comprise obtaining the status of an imaging trigger means (e.g. pedal or button), wherein the guidelines are only displayed if the imaging trigger means is activated.

The reference image and the live image may be displayed side by side, wherein the guideline is a horizontal guideline, or the reference image and the live image may be displayed one above the other, wherein the guideline is a vertical guideline. By "horizontal" is meant the row direction of a display, and by "vertical" is meant the column direction of a display.

The reference image and the live image may be displayed at the same size.

Displaying the reference image and the live image at the same size provides a one-to-one mapping between both images which makes it easier for the user to determine the correspondence between the live image and the reference image.

The reference image may be a static treatment planning image. The live medical image may be an X-ray image. Other types of live medical images may be used provided that they can be aligned with the reference image.

The invention also provides a computer program product comprising computer program code which, when executed on a computing device having a processing system, cause the processing system to perform all of the steps of the afore-mentioned method.

The invention also provides a system for displaying live medical images of a subject, the system comprising:
a display for displaying images to a user; and
a processor configured to:
   receive a reference medical image and a live medical image of the subject, wherein the reference image contains at least one marked position;
   obtain the angle and magnification characteristics of the reference image and the live image;
   verify whether the reference image and the live image are aligned based on their angle and magnification characteristics; and
   if the reference image and the live image are aligned:
      display, on the display, the reference image and the live image; and
      overlay at least one guideline on top of the reference image and the live image, wherein the guideline crosses the at least one marked position on the reference image.

If the reference image and the live image do not align, the processor may be further configured to notify the user that the images do not align and/or provide the user with an option to perform realignment of the live image with the reference image.

The processor may be further configured to display, on the display, a plurality of co-registration images and obtain a user selected reference image from the plurality of co-registration images.

The system may further comprise an imaging trigger means (e.g. pedal, button, UI tab) wherein the processor is further configured to obtain the status of the imaging trigger means, and wherein the guidelines are only displayed if the imaging pedal is pressed.

The processor may be configured to display the reference image and the live image side by side, wherein the guideline is a horizontal guideline, or display the reference image and the live image one above the other, wherein the guideline is a vertical guideline.

The reference image may be a static treatment planning image and the live medical image may be an X-ray image.

The processor may be configured to display the reference image and the live image at the same size.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for displaying live medical images concurrently with a reference image;
Figure 2 illustrates a live imaging system;
Figure 3 illustrates a first embodiment in which the method for displaying live images concurrently with reference images may be used; and
Figure 4 illustrates a second embodiment in which the method for displaying live images concurrently with reference images may be used.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

The invention provides a computer-implemented method for displaying live medical images of a subject. The method comprises receiving a reference medical image and a live medical image of the subject. The reference image contains at least one marked position. Angle and magnification (i.e. zoom) characteristics of the reference image and the live image are obtained. The method further comprises checking whether the reference image and the live image align based on their angle and zoom characteristics. If the reference image and the live image align, the reference image and live image are displayed to the user and at least one guideline is overlaid on top of the reference image and the live image. The guideline crosses the at least one marked position on the reference image. The region of interest identification and indication in live medical images is improved based on which the physician can plan a treatment with high confidence. Such treatment may be an apposition of a tent at a certain location that is determined in the reference image at the marked position and which stent apposition is effectively appositioned with the use of live medical image, such as for example fluoroscopy by using the guideline or guidelines indicating the positions in the live medical images that correspond to the marked position in the reference image. Other such treatments that benefit the invention are: angioplasty at a predetermined location; placement of various implants at predetermined locations such as filters in vessels or valves in heart; removal or treatment of lesions in vessels by mechanical (e.g. atherectomy), optical or release of chemical agents at predetermined location; navigating guidewires for diagnostic measurements, accessing vessel branches; entry or exit points to or from anatomic structures at predetermined position for example oncology treatment or renal denervation, etc.

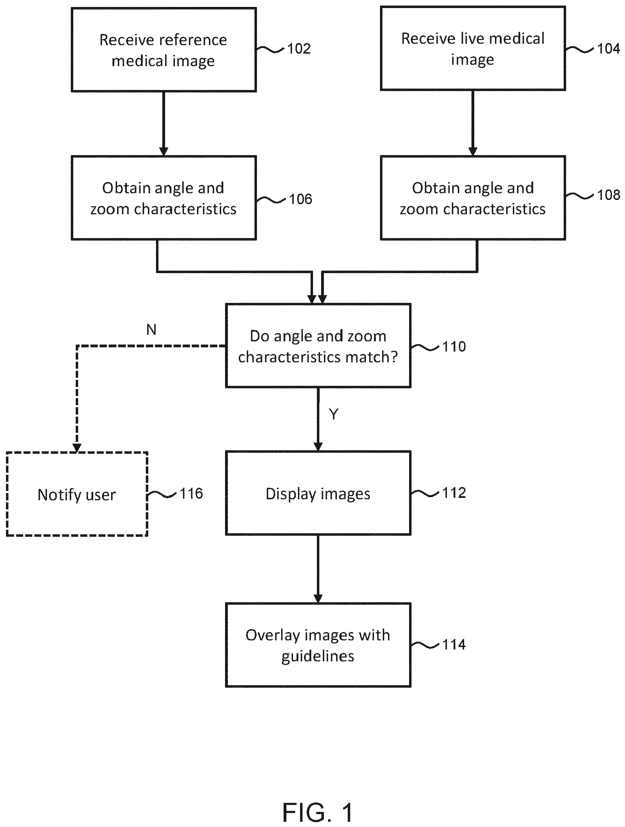

Figure 1 shows a method for displaying live medical images concurrently with a reference image. The method comprises obtaining live medical images in step 104 and obtaining a reference medical image in step 102. The live medical images may be live X-ray feed of images such as fluoroscopy images. Other live medical images could also be used such as provided by magnetic resonance imaging (MRI), ultrasound imaging, positron emission tomography imaging (PET), single-photon emission computerized tomography (SPECT) etc. as long as a region of interest of the subject is visible (internal and/or external).

The reference image may be a treatment planning image. The treatment planning image may contain details of the subject's anatomy near the region of interest. In general, any reference image will contain at least one marked position. The marked position is indicative of an important position on the region of interest. The marked position in a treatment planning image may be a position on the region of interest relevant to a particular treatment (e.g. a target position for a guidewire).

The method also comprises obtaining zoom and angle characteristics of the live medical image and the reference image in steps 106 and 108 respectively. The zoom characteristics of the live medical image may include a zoom factor of the imaging device used to obtain the live medical images. The angle characteristics of the live medical image may include the angulation of the imaging device used to obtain the live medical images.

The method then comprises checking if the reference image's zoom and angle characteristics match the live medical image's angle and zoom characteristics in step 110. Checking if the angle and zoom characteristics match may comprise using a calibration between the angle and zoom characteristics of the live medical images and the reference image. If the reference image was obtained using the same imaging device as the live medical images, there may be no need to perform a calibration. Checking if the angle and zoom characteristics match will be further explained below.

When the zoom and angulation align, the live medical images and the reference image are displayed such that they align with each other in step 112. For example, the live medical images may be displayed side by side and at the same size. Guidelines are overlaid on top of both the live medical images and the reference image such that they cross the marked position(s) on the reference image in step 114. For example, if the live medical images and the reference image are displayed side by side, horizontal guidelines are used.

When the zoom and angulation misalign, the user may be notified that the angle and zoom characteristics don't align in step 116. Additionally, the user may be notified whether it is the angle characteristics and/or the zoom characteristics that misalign and by how much. The user may be notified that the horizontal guides are not being shown because the live images don't align with the reference image.

Since the system knows the angle and zoom characteristics of the reference image, besides notifying the user, the system may also provide an option according to which the user is able to realign the system to match with the reference image characteristics (instead of manually adjusting these characteristics), thereby ensuring that there is no offset. This may for example be realized by providing a user interface (UI) "Accept" button.

This option will automatically re-align the imaging sensor to the angle and zoom characteristics of the reference image. Re-aligning the image sensor to the characteristics of the reference image may comprise moving/rotating the image sensor such that the live medical images it obtains have angle and zoom characteristics which match the reference image's characteristics. It may additionally or alternatively comprise adapting the zoom of the live medical images or the reference medical image in software and/or hardware.

Additionally, the live images and reference image may be shown side by side, with no guidelines, in the same size even if they do not align. Doing so, may make it easier for the user to align markings/regions on the reference image with the data on the live images using the bare eye. Displaying the guidelines only when the live images align with the reference image provides a clear indication to the user that the images align without needing to look away from the live images.

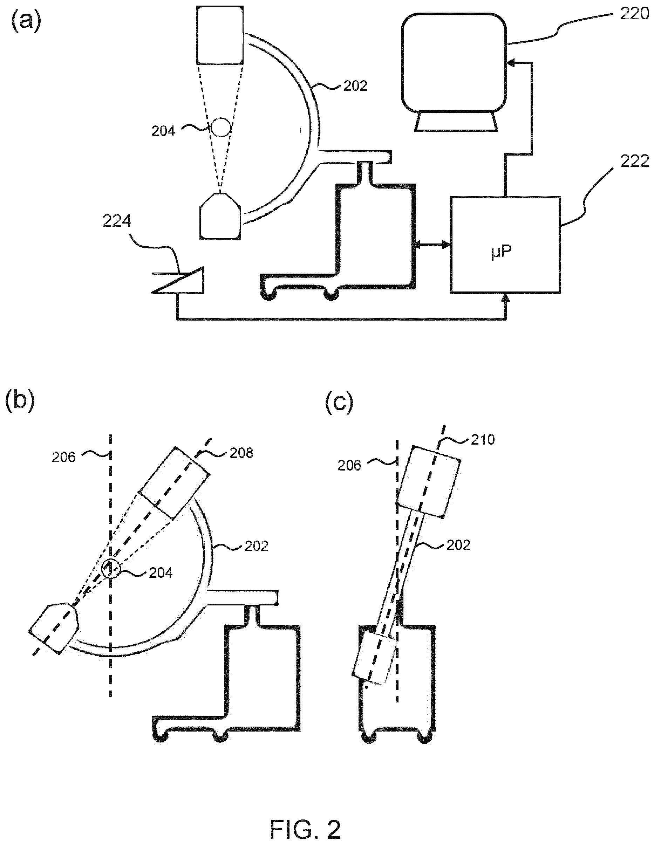

Figure 2 illustrates a live imaging system. The live imaging system comprises a C-arm X-ray device 202 with an X-ray source at the upper portion of the C-arm 202 and an X-ray detector at the lower portion of the C-arm 202. An object of interest 204 (e.g. a subject) is placed between the C-arm 202 such that it can be imaged by the C-arm X-ray device.

Figure 2 (a) shows the C-arm 202 in a vertical position. Figure 2(a) also shows a display 220 for displaying the images and a processor 222 for controlling the display 220. An imaging pedal 224 is also shown.

Figure 2 (b) shows the C-arm 202 after a first rotation. For clarity, the display, processor and imaging pedal are not shown. The first rotation changes the angulation of the X-ray images obtained. The angle of rotation, from the C-arm 202 of Figure 2 (a) to the C-arm 202 of Figure 2 (b), is illustrated by the angle between a vertical, reference, axis 206 and the new imaging axis 208. The first angle of rotation could be measured by the particular position of the C-arm 202. The first rotation axis is a first horizontal axis.

Similarly, Figure 2 (c) shows the C-arm 202 after a second rotation. The second rotation is around a second rotation axis which is perpendicular to the axis of rotation for the first rotation, but is again horizontal. The second rotation axis is thus a second horizontal axis perpendicular to the first horizontal axis. The angle of rotation, between reference axis 206 and new imaging axis 210, due to the second rotation also forms part of the angle characteristics. The particular form of the angle characteristics may depend on the degrees of freedom of the particular imaging system and may contain one or more angles.

The angle and zoom characteristics may be of the form [ *θₓ , θ_{y}* , *θ_{z} , Z* ] where the first three parameters define three angles of rotation relative to a reference axis 206 and the fourth parameter defines the zoom (e.g. Z = 110% would indicate a 10% magnification over the original live image).

Preferably, the live imaging system stores the angle characteristics for every frame of the live images. Some live imaging systems include angle and zoom characteristics in the corresponding data of the live images. For other live imaging systems, other means may be needed to send angle and zoom characteristics to an external processor. The zoom characteristics may form part of the user interface and the user may be able to select a zoom factor for the live images and the reference image. In some cases, the zoom characteristics may factor in the distance between the imaging sensor and the subject being imaged.

In the case where the live images are obtained with the same imaging device as the reference images, the angle and zoom characteristics of the live images can be compared directly to the angle and zoom characteristics of the reference image. However, in the case where live images are obtained with a different imaging device and/or the zoom and angle characteristics do not correspond, it may be necessary to calibrate the zoom and angle characteristics between the live images and the reference image.

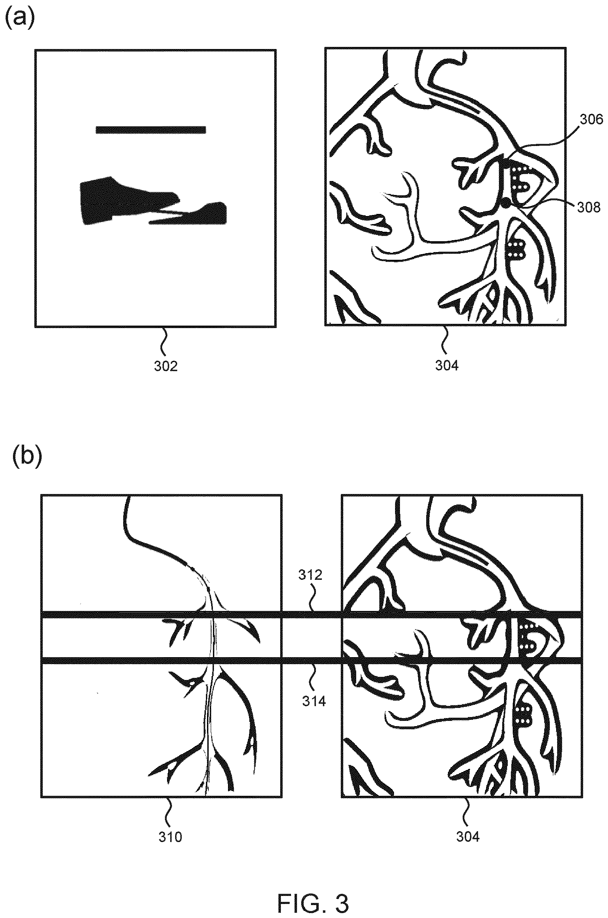

Figure 3 illustrates a first scenario in which the method for displaying live images concurrently with reference images may be used. Figure 3 (a) illustrates a display before a live X-ray feed, i.e. fluoroscopy, is activated. The image 302 notifies the user that there is no live X-ray feed and that the X-ray pedal needs to be pressed in order to activate the live X-ray feed. Other means of activating or triggering the live X-ray feed could also be used, such as for example a physical button or a particular area of a touch user interface (e.g. mouth button, tab on a touch display).

A reference image 304 is also displayed. In this case, the reference image comprises two marker points 306 and 308 at points of interest on the anatomy of the subject. The user may have access to a panel from which they can select a reference image from a set of reference images. The set of reference images may have different types of angle and/or zoom characteristics.

Characteristics such as angle are typically set before the X-ray pedal is pressed and not while the pedal is pressed. The zoom characteristics are typically applied using a user interface to zoom in on particular details or zoom out to see the images in their original dimensions, i.e. no magnification and no demagnification. Thus, prior to the user activating the live imaging (e.g. by pressing the X-ray pedal), the user could receive a notification that the angle and zoom characteristics of the live images will match the angle and zoom characteristics of the selected reference image. The user could also be notified of which reference images, in the set of reference images, have matching angle and zoom characteristics.

The user interface may also give the user the option to only show the reference images in the panel which match the current angle of the C-arm position. The zoom characteristics can be matched before or after activating the live imaging. However, for X-ray imaging, it is typically preferable to reduce the time during which the subject is exposed to X-ray radiation. Thus, the user interface preferably enables the user to align the reference images to the angulation of the C-arm and match the zoom factors prior to activating the live imaging.

If the user attempts to select a reference image that does not align with current C-arm position, the user may be notified that the live images will not align with the live images.

Figure 3 (b) shows a display after the live imaging is activated. In this case, this occurs after the user presses the X-ray pedal as requested in image 302. The live X-ray, fluoroscopy images 310 appear as soon as the X-ray pedal is pressed. The live X-ray images 310 show a live feed of the subject's anatomy at the region of interest. The reference image 304 and the fluoroscopy images 310 are aligned and displayed side by side at the same size. In an alternative embodiment the latest available frame of the X-ray images 310 is displayed.

The two marker points 306 and 308 shown in Figure 3 (a) are detected on the reference image 304. This triggers the projection of two horizontal guidelines 312 and 314 that cross the marker points and overlay the two images, thereby extending the y position of the markers to the live X-ray image, with other words the guidelines stretch through the marker points across the reference and fluoroscopy images. The user can now use the horizontal guidelines 312 and 314 to position a therapeutic device at the planned position.

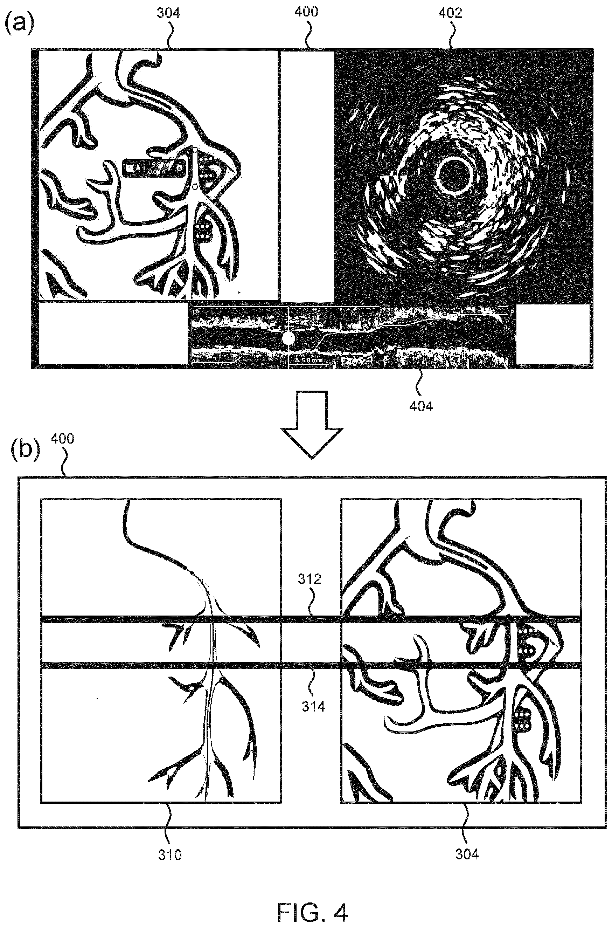

Figure 4 illustrates a second embodiment in which the method for displaying live images with reference images may be used concurrently. The imaging system in this example may comprise a co-registration feature. Figure 4 (a) shows an exemplary display 400 with the co-registration feature. This feature displays a co-registration of angiographic images with intravascular ultrasound (IVUS) imaging results or physiology images, e.g. fractional flow reserve (FFR), instantaneous Flow reserve (iFR), flow velocity. For example, IVUS co-registration is a feature that enables the user to accurately map an IVUS image 402 to a specific location along an angiographic image as well as showing a vessel reconstruction 404 obtained from the IVUS images over time. In this case, the angiographic image is a reference image 304 used by the user to plan the treatment.

Seeing the IVUS results 402 and 404 in conjunction with the reference image 304 helps the user identify and determine a region of interest (e.g. determine where to place a stent, the length of stent required, the diameter stent required etc.) and effectively plan the treatment. Thus, the IVUS co-registration may help the user determine where to place the marker points as part of the treatment planning.

After the treatment planning, the user may commence the treatment (e.g. placing a stent on the marker points) with live X-ray imaging. As with the first scenario, the user may be notified of whether the angle and zoom characteristics of the live imaging device match the angle and zoom characteristics of the reference image annotated with the marker points during the treatment planning.

Figure 4 (b) shows the display 400 when live X-ray imaging is activated. When the user presses the X-ray pedal, the live X-ray images 310 and reference image 304 are shown side by side at the same size. Two marker points on the reference image are detected and two horizontal guidelines 312 and 314 are projected on the images, where the guidelines 312 and 314 cross the marker points on the reference image 304 and overlay both the live images 310 and the reference image 304, thereby extending the y position of the marker points to the live X-ray images 310.

The user can now use the horizontal guidelines to position a therapeutic device (e.g. stent) at the planned position(s) determined with the IVUS co-registration feature during the treatment planning phase. When the user releases the foot from the X-ray pedal or deactivates the fluoroscopy imaging trigger, the overlay may disappear automatically.

In cases where the user needs to put multiple stents (e.g. where two stents might be perpendicular to each other), it may be preferable to display the live images directly above or below the reference images. If the two or more marker points are in similar y positions of the reference image, wherein y denotes the height of the position in the image, it may also be preferable for the images to be placed one above the other instead of side by side such that the guidelines can be clearly be distinguished from each other. Having both vertical and horizontal guidelines on the live images, and therefore two copies of the reference image, may also be possible.

The skilled person would be readily capable of developing a processor for carrying out any herein described method. Thus, each step of a flow chart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for displaying live medical images of a subject, the system comprising:
a display (220) for displaying images to a user; and
a processor (222) configured to:
receive a reference medical image and a live medical image of the subject, wherein the reference image comprises at least one marked position;
obtain angle and magnification characteristics of the reference image and the live image;
check whether the angle and magnification characteristics of the reference image and the live image match; and
if the match is confirmed:
display, on the display, the reference image and the live image; and
overlay at least one guideline on top of the reference image and the live image, wherein the at least one guideline passes through the respective at least one marked position on the reference image.

2. The system of claim 1, wherein, if the match is not confirmed, the processor is further configured:
to notify the user that the images do not align.

3. The system of claim 1 or 2, wherein the processor is further configured to provide the user with an option to perform realignment of the live image with the reference image in order to bring the angle and magnification characteristics of the reference image and the live image to match.

4. The system of any one of claims 1 to 3, wherein the processor is further configured to:
display, on the display, a plurality of co-registration images; and
wherein the reference image is a user selected image from the plurality of co-registration images.

5. The system of any one of claims 1 to 4, further comprising an imaging trigger means (224), wherein the processor is further configured to obtain the status of the imaging trigger means, wherein the at least one guideline is solely displayed during activation of the imaging trigger means.

6. The system of any one of claims 1 to 5, wherein the processor is configured to:
display the reference image and the live image side by side and the guideline is horizontal; or
display the reference image and the live image one above the other and the guideline is vertical.

7. The system of any one of claims 1 to 6, wherein the reference image is a static treatment planning image and the live medical image comprises fluoroscopy images.

8. A computer-implemented method for displaying live medical images of a subject, the method comprising:
(102) receiving a reference medical image and a live medical image of the subject, wherein the reference image comprises at least one marked position;
(106) obtaining angle and magnification characteristics of the reference image and the live image;
(110) checking whether the reference image and the live image align based on their angle and magnification characteristics; and
if the reference image and the live image align:
(112) displaying, to a user, the reference image and the live image; and
(114) overlaying at least one guideline on top of the reference image and the live image, wherein the at least one guideline crosses the respective at least one marked position on the reference image.

9. The method of claim 8, wherein, if the reference image and the live image do not align, the method further comprises:
notifying the user that the images do not align; and/or
providing the user with an option to perform realignment of the live image with the reference image.

10. The method of claim 8 or 9, further comprising:
displaying a plurality of co-registration images to the user; and
obtaining a user selected reference image from the plurality of co-registration images.

11. The method of any one of claims 8 to 10, further comprising obtaining the status of an imaging trigger means, wherein the at least one guideline is only displayed when the imaging trigger means is activated.

12. The method of any one of claims 8 to 11, wherein:
the reference image and the live image are displayed side by side and the guideline is horizontal; or
the reference image and the live image are displayed one above the other and the guideline is vertical.

13. The method of any one of claims 8 to 12, wherein the reference image and the live image are displayed at the same size.

14. The method of any one of claims 8 to 13, wherein the reference image is a static treatment planning image and the live medical image comprises fluoroscopy images.

15. A computer program product comprising computer program code which, when executed on a computing device having a processing system, cause the processing system to perform all of the steps of the method according to any of claims 8 to 14.
